# FASCICULE DE BREVET EUROPEEN

(11) **EP 2 520 333 B1**
(45) Date de publication et mention de la délivrance du brevet: **03.09.2014**
(21) Numéro de dépôt: 12164390.2
(22) Date de dépôt: 17.04.2012
(51) Int. Cl.: A61N 1/378

(54) **Dispositif de récupération d'énergie pour capsule intracorporelle autonome**
Vorrichtung zur Energierückgewinnung für autonome intrakorporelle Kapsel
Energy recovery device for autonomous intracorporeal capsule

(30) Priorité: 04.05.2011 FR 1153790
(43) Date de publication de la demande: 07.11.2012
(73) Titulaire: Sorin CRM SAS, 92140 Clamart Cedex (FR)
(72) Inventeur: Deterre, Martin, 75013 Paris (FR); Lefeuvre, Elie, 93100 Montreuil (FR)
(74) Mandataire: Dupuis-Latour, Dominique

(56) Documents cités:
- US-A1- 2007 167 988
- US-A1- 2009 216 292
- US-A1- 2009 240 299
- US-A1- 2010 160 994

## Description

L'invention concerne, de façon générale, le domaine des "dispositifs médicaux actifs" tels que définis par la directive 93/42/CE du 14 juin 1993 du Conseil des communautés européennes, et notamment les "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resynchronisation, de défibrillation et/ou de cardioversion en cas de trouble du rythme détecté par l'appareil. Elle inclut aussi les appareils neurologiques, les implants cochléaires, etc., ainsi que les dispositifs de mesure de pH ou encore d'impédance intracorporelle (telle que mesure d'impédance trans-pulmonaire ou d'impédance intracardiaque).

L'invention concerne plus particulièrement ceux de ces dispositifs qui mettent en oeuvre des capsules autonomes implantées et dépourvues de toute liaison physique à un dispositif principal implanté (tel qu'un boîtier de générateur d'impulsions de stimulation).

Ces capsules autonomes sont dénommées pour cette raison "capsules *leadless",* pour les distinguer des électrodes ou des capteurs disposés à l'extrémité distale d'une sonde (*lead*)*,* cette sonde étant parcourue sur toute sa longueur par un ou plusieurs conducteurs reliant par voie galvanique l'électrode ou le capteur à un générateur connecté à l'extrémité opposée, proximale, de la sonde.

De telles capsules *leadless* sont par exemple décrites dans les US 2007/0088397 A1 et WO 2007/047681 A2 (Nanostim, Inc.) ou encore dans le US 2006/0136004 A1 (EBR Systems, Inc.).

Ces capsules *leadless* peuvent être par exemple des capsules épicardiques, fixées à la paroi extérieure du coeur, ou bien des capsules endocavitaires, fixées à la paroi intérieure d'une cavité ventriculaire ou auriculaire au moyen d'une vis d'ancrage saillante, prolongeant axialement le corps de la capsule et destinée à pénétrer dans le tissu cardiaque par vissage au site d'implantation.

Une telle capsule comprend des circuits de détection/stimulation pour recueillir des potentiels de dépolarisation du myocarde et/ou pour appliquer des impulsions de stimulation au site où est implantée la capsule. La capsule porte alors une électrode appropriée, qui peut être notamment constituée par une partie active de la vis d'ancrage.

Elle peut également incorporer, en variante ou en complément, un ou plusieurs capteurs permettant de mesurer localement la valeur d'un paramètre tel que le niveau d'oxygène dans le sang, la pression cardiaque endocavitaire, l'accélération de la paroi cardiaque, l'accélération du patient comme indicateur de l'activité, etc. Bien entendu, les capsules *leadless* incorporent également des moyens émetteurs/récepteurs de communication sans fil pour l'échange de données à distance.

L'invention n'est toutefois pas limitée à un type particulier de capsule, et elle est applicable indifféremment à tout type de capsule *leadless,* quelle que soit sa destination fonctionnelle.

Quelle que soit la technique mise en oeuvre, le traitement des signaux au sein de la capsule et leur transmission à distance nécessite une énergie non négligeable par rapport aux ressources énergétiques que peut stocker cette capsule. Or, compte tenu de son caractère autonome, la capsule ne peut faire appel qu'à ses ressources propres telles qu'un circuit de récupération d'énergie (par le mouvement de la capsule), associé à une petite batterie tampon intégrée.

La gestion de l'énergie disponible est donc un point crucial pour le développement des techniques de capsules autonomes *leadless,* en particulier leur aptitude à disposer d'un système d'auto-alimentation intégré.

De nombreuses techniques de récupération d'énergie ont été proposées, adaptées à des implants autonomes de type *leadless.*

Ainsi, les US 2006/0217776 A1, US 3 456 134 A et WO 2007/149462 A2 décrivent des systèmes utilisant des transducteurs piézoélectriques transformant directement en énergie électrique les déplacements d'une masse sous l'effet de l'accélération des organes ou du corps du patient. Toutefois, compte tenu des fréquences d'excitation relativement basses (inférieures à 10 Hz), les excursions de ces déplacements sont relativement importantes, ce qui ne permet pas d'atteindre une miniaturisation poussée. De plus, comme ces excitations ne présentent pas de fréquences spécifiques stables, le générateur piézoélectrique ne peut pas fonctionner en mode résonant, et perd de ce fait beaucoup de son efficacité.

D'autres dispositifs proposent de transformer en électricité des variations de pression apparaissant au sein du corps, notamment les variations de pression sanguine ou celles résultant des mouvements du diaphragme pendant la respiration. Cette transformation est opérée au moyen d'un microgénérateur magnétique, fonctionnant à la manière d'un alternateur ou d'une dynamo, par des variations de flux magnétique induites dans un bobinage. On pourra se référer notamment aux US 2005/0256549 A1, GB 2 350 302 A, US 2008/0262562 A1 ou US 2007/0276444 A1. Du fait de la présence de pièces mobiles, la complexité de l'agencement des pièces mécaniques et électriques et leur volume relativement important limitent cependant largement les possibilités de miniaturisation ainsi que la fiabilité d'ensemble d'un tel générateur. En outre et surtout, un tel générateur est par nature sensible aux champs magnétiques externes et n'est donc pas compatible avec les systèmes d'imagerie par résonance magnétique (IRM) du fait des champs magnétiques statiques très élevés générés par ces systèmes, typiquement de l'ordre de 0,5 à 3 T, voire plus.

Il a également été proposé d'utiliser un transducteur électrostatique constitué d'électrodes modélisant un condensateur, par exemple avec un ensemble de peignes et contre-peignes interdigités. L'une des électrodes est solidaire d'un support fixé au corps du boîtier, l'autre étant couplée à une masse oscillante dite "masse sismique". Cette masse est mise en mouvement par les déplacements d'ensemble du dispositif intégrant le transducteur, et elle entraîne avec elle l'une des électrodes du transducteur, qui va ainsi se mouvoir par rapport à l'autre en faisant varier l'intervalle diélectrique et/ou la surface en regard des deux électrodes.

Si le condensateur a été initialement préchargé, ou si la structure porte des électrets permettant de conserver une charge permanente, la variation de capacité provoque dans ce condensateur une augmentation d'énergie qui peut être extraite par un circuit électronique et stockée ensuite dans une batterie tampon. L'énergie mécanique recueillie par la masse oscillante peut être ainsi convertie quasiment en totalité en énergie électrique, en un seul cycle.

Cette technique est par exemple décrite par F. Peano et T. Tambosso, Design and Optimization of a MEMS Electret-Based Capacitive Energy Scavenger, Journal Of Microelectromechanical Systems, 14(3), 429-435, 2005, ou encore par S. Meninger et al. Vibration-to-Electric Energy Conversion, IEEE Transactions on Very Large Scale Integration (VLSI) Systems, vol. 9, no. 1, pp. 64-76, 2001.

Ce type de transducteur présente toutefois les mêmes inconvénients que les transducteurs piézoélectriques du fait des limitations imposées par la masse oscillante, à la fois en termes de miniaturisation (la masse sismique est relativement volumineuse) et d'efficacité à l'égard des mouvements d'entraînement. En effet, les fréquences d'excitation relativement basses (inférieures à 10 Hz) impliquent des excursions relativement importantes et/ou une masse de l'élément oscillant relativement élevée, ce qui ne permet pas d'atteindre une miniaturisation poussée.

Le but de l'invention est de proposer un générateur d'alimentation électrique pour une capsule autonome implantable qui pallie les inconvénients ci-dessus et réponde aux impératifs de :
- miniaturisation : compatibilité avec le volume extrêmement réduit (de quelques millimètres cubes) d'un implant *leadless* ;
- fiabilité : garantie d'un fonctionnement assuré sur plusieurs années de durée de vie de l'implant ;
- insensibilité aux phénomènes magnétiques : notamment pour assurer la compatibilité IRM qui est aujourd'hui requise pour les dispositifs implantés ; et
- biocompatibilité : absence d'éléments extérieurs risquant de provoquer des réactions inflammatoires.

Un autre système de récupération d'énergie, dépourvu de masse oscillante, est divulgué par le US 2009/021292 A1. Ce document propose un système d'alimentation par récupération d'énergie incorporé à une capsule implantable dont le corps de boîtier possède un élément déformable sous l'effet des variations de pression du milieu environnant.

La déformation de cet élément est transmise à un transducteur électrostatique convertissant directement l'énergie mécanique de déformation en énergie électrique, qui est ensuite délivrée à un module de gestion électrique et de stockage alimentant l'implant en énergie.

On notera que le système n'a besoin ni d'être résonant ni de contenir des éléments magnétiques.

Ce document décrit toutefois un système dans lequel les variations de pression résultent au moins partiellement d'efforts mécaniques appliqués à la capsule, sous l'effet de forces de contact avec les tissus environnants ou de déformations de ceux-ci.

Dans le cas d'un système totalement immergé dans un fluide corporel (comme par exemple pour une capsule intracardiaque devant récupérer l'énergie à partir des variations de pression du sang lors des variations rapides du cycle diastole-systole), les variations lentes de la pression atmosphérique viennent perturber le fonctionnement du système : en effet, comme la capsule est strictement étanche, son volume intérieur se trouve initialement à la pression définie en usine et le point d'équilibre au repos de l'élément déformable va se trouver décalé par rapport à la position de repos nominale si la pression atmosphérique varie.

Le problème de l'invention est d'éliminer ce biais perturbateur, pour maximiser la récupération d'énergie en faisant en sorte que les variations du cycle cardiaque diastole-systole soient intégralement transmises aux électrodes autour du même point de repos nominal, le récupérateur pouvant ainsi jouer complètement le rôle qui lui a été assigné.

Plus précisément, la présente invention concerne une capsule intracorporelle autonome d'un type comparable à celui décrit dans le US 2009/021292 A1 précité, c'est-à-dire comportant un corps et, à l'intérieur de ce corps, des circuits électroniques et des moyens d'alimentation électrique comprenant :
- un transducteur de récupération d'énergie, apte à convertir une sollicitation physique extérieure appliquée à la capsule en une grandeur électrique, ce transducteur comprenant :
   - une première électrode de condensateur, couplée à un élément mobile d'actionnement recevant ladite sollicitation physique extérieure, l'élément mobile d'actionnement du transducteur étant essentiellement dépourvu de masse oscillante et comprenant une surface déformable, formée sur l'extérieur du corps de la capsule et apte à être déformée alternativement dans un sens et dans l'autre sous l'effet des variations de pression dans le milieu environnant la capsule. et
   - une seconde électrode de condensateur, montée sur un support relié à une région du corps autre que l'élément mobile d'actionnement, les deux électrodes présentant des surfaces en vis-à-vis séparées par un intervalle diélectrique définissant ensemble un condensateur, et ladite sollicitation physique produisant une modification corrélative desdites surfaces en vis-à-vis et/ou dudit intervalle diélectrique avec variation corrélative de la capacité dudit condensateur ; et
- un module de stockage et de gestion d'énergie, alimenté par le transducteur de récupération d'énergie sous l'effet d'une diminution des surfaces en vis-à-vis et/ou d'une augmentation de l'intervalle diélectrique du condensateur,

Les deux électrodes présentent des surfaces en vis-à-vis séparées par un intervalle diélectrique définissant ensemble un condensateur, et la déformation de la surface déformable produit une modification corrélative desdites surfaces en vis-à-vis et/ou dudit intervalle diélectrique avec variation corrélative de la capacité du condensateur. Par ailleurs, le module de gestion comprend des moyens aptes à précharger ledit condensateur lorsque sa capacité est maximale, et à décharger celui-ci en transférant son énergie vers les moyens de stockage lorsque diminue cette capacité sous l'effet d'une diminution des surfaces en vis-à-vis et/ou d'une augmentation de l'intervalle diélectrique du condensateur.

De façon caractéristique de l'invention, la surface déformable est couplée à la première électrode avec interposition d'un élément amortisseur mécanique formant filtre passe-haut à l'égard des variations de pression dans le milieu environnant la capsule.

La surface déformable peut comprendre une surface rigide couplée à la première électrode et un organe élastiquement déformable de liaison de cette surface rigide au reste du corps, ou bien une membrane couplée à la première électrode dans une région de plus grande déformation de celle-ci.

Les première et seconde électrodes sont avantageusement réalisées sous forme de peignes et contre-peignes interdigités, et la première peut être couplée au corps de la capsule par un support élastiquement déformable formant ressort de guidage.

La capsule peut en outre comprendre des moyens aptes à précharger le condensateur lorsque sa capacité est maximale, et à décharger celui-ci en transférant son énergie vers les moyens de stockage lorsque diminue cette capacité sous l'effet d'une diminution des surfaces en vis-à-vis et/ou d'une augmentation de l'intervalle diélectrique du condensateur.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 illustre de façon schématique un ensemble de dispositifs médicaux comprenant notamment des capsules *leadless,* implantées au sein du corps d'un patient.
La Figure 2 est un schéma par blocs fonctionnels montrant les différents étages constitutifs d'une capsule *leadless.*
Les Figures 3 et 4 illustrent deux formes de réalisation possibles du corps de la capsule implantable selon l'invention.
Les Figures 5a et 5b sont des vues schématiques en coupe d'un premier mode de réalisation d'une capsule à récupération d'énergie par voie électrostatique, montrant les principes mis en oeuvre.
La Figure 6 est une vue en plan, en coupe selon VI-VI de la Figure 5a, d'un premier mode de réalisation d'une capsule à récupération d'énergie par voie électrostatique.
La Figure 7 est un diagramme charge/tension montrant deux manières d'opérer la récupération d'énergie, à charge constante ou à tension constante.
La Figure 8 est une représentation schématique d'un circuit de récupération d'énergie à tension constante.
La Figure 9 est une représentation schématique d'un circuit de récupération d'énergie à charge constante.
La Figure 10 illustre un deuxième mode de réalisation d'une capsule à récupération d'énergie par voie électrostatique, permettant une récupération d'énergie dans les deux sens de déplacement de l'électrode mobile.
La Figure 11 illustre un troisième mode de réalisation d'une capsule à récupération d'énergie par voie électrostatique, comportant un empilement de structures telles que celle illustrée Figure 5a pour le premier mode de réalisation.
La Figure 12 illustre un quatrième mode de réalisation d'une capsule à récupération d'énergie par voie électrostatique, dans lequel l'élément déformable est une membrane souple.
La Figure 13 illustre un cinquième mode de réalisation d'une capsule à récupération d'énergie par voie électrostatique, comprenant un soufflet de liaison de l'élément mobile au corps de la capsule.
La Figure 14 illustre le perfectionnement selon l'invention, applicable aux divers modes de réalisation présentés plus haut, permettant d'éliminer les effets des variations lentes de pression.
La Figure 15 illustre une réalisation particulière d'une capsule à récupération d'énergie par voie électrostatique, en technologie photolithographique avec des peignes fixes et des contre-peignes mobiles suspendus par des ressorts de guidage réalisés également par photogravure.
Les Figures 16 et 17 illustrent deux variantes de réalisation de la structure de condensateur, adaptées notamment à l'utilisation d'armatures porteuses d'électrets.

On va tout d'abord décrire divers exemples de réalisation d'une capsule à récupération d'énergie par voie électrostatique.

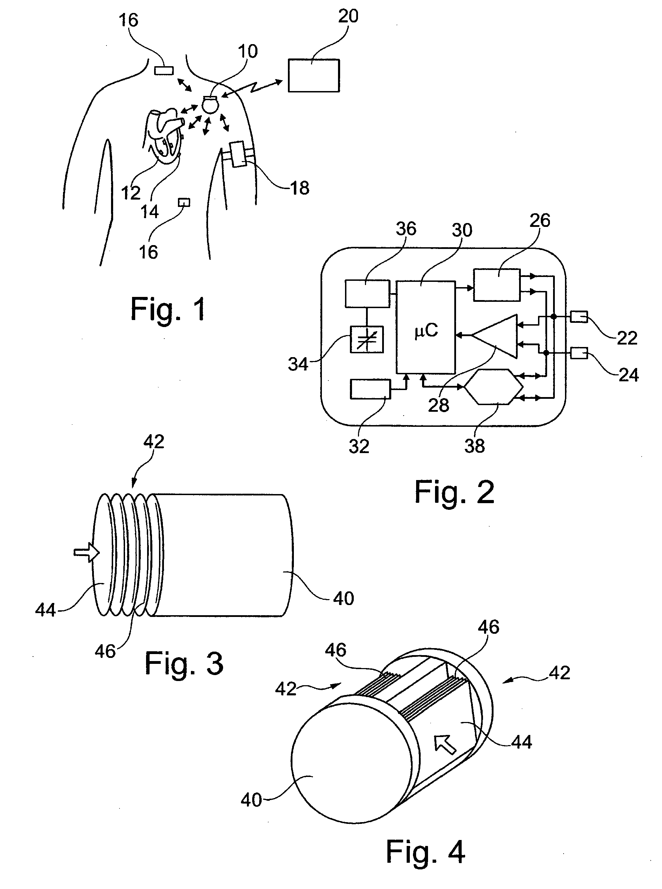

Sur la Figure 1 on a illustré un ensemble de dispositifs médicaux implantés au sein du corps d'un patient.

Celui-ci est équipé par exemple d'un implant 10 tel qu'un défibrillateur/stimulateur/resynchroniseur implanté, un défibrillateur sous-cutané ou encore un enregistreur longue durée. Ce dispositif 10 est le dispositif maître d'un réseau comportant une pluralité de dispositifs esclaves 12 à 18, qui peuvent notamment inclure des capsules intracardiaques 12 ou épicardiques 14 implantées directement sur le coeur du patient, d'autres dispositifs 16 tels que capteurs de myopotentiels ou dispositifs de stimulation neurologique, et éventuellement un dispositif externe 18 disposé sur un brassard et pourvu d'électrodes en contact avec la peau. Le dispositif 10 peut également être utilisé en tant que passerelle avec le monde extérieur pour communiquer avec un périphérique externe 20 du type programmateur ou dispositif de télétransmission de données avec lequel il pourra communiquer par télémétrie.

La Figure 2 illustre de façon schématique les différents circuits internes des capsules autonomes implantées 12 à 16.

La capsule comporte par exemple un couple d'électrodes 22, 24 reliées à un circuit 26 générateur d'impulsions de stimulation (pour une capsule active incorporant cette fonction) et/ou à un circuit de détection 28 servant au recueil des potentiels de dépolarisation recueillis entre les électrodes 22 et 24. Un circuit central 30 inclut l'ensemble de l'électronique permettant de piloter les diverses fonctions de la capsule, la mémorisation des signaux recueillis, etc. Il comprend un microcontrôleur et un oscillateur générant les signaux d'horloge nécessaires au fonctionnement du microcontrôleur et à la communication. Il peut également contenir un convertisseur analogique/numérique et une mémoire de stockage numérique. La capsule peut également être pourvue d'un capteur 32 tel qu'un capteur d'accélération, de pression, un capteur hémodynamique, de température, de saturation en oxygène, etc. La capsule comprend un étage de récupération d'énergie 34 alimentant l'ensemble des circuits via un étage de gestion d'énergie 36. Les électrodes 22 et 24 sont également reliées à un circuit d'émission/réception d'impulsions 38 servant à la communication sans fil avec le dispositif maître ou les autres capsules.

L'invention concerne plus particulièrement l'étage de récupération d'énergie 34 qui, de façon caractéristique, utilise les variations de pression du milieu environnant, notamment les variations cycliques de la pression sanguine, pour déplacer une électrode d'un élément de condensateur par rapport à une autre électrode en vis-à-vis. La récupération d'énergie est obtenue grâce à la variation de capacité du condensateur résultant du déplacement relatif des deux électrodes, qui entraîne une modification de leurs surfaces en regard et/ou une variation de l'intervalle diélectrique qui les sépare.

Pour pouvoir prendre en compte ces déformations, la capsule est réalisée sous forme d'un corps 40 pourvu, comme illustré Figures 3 et 4, d'un ou plusieurs éléments déformables 42 sollicités au rythme des variations de la pression du fluide dans lequel baigne la capsule (typiquement les variations de pression sanguine, dans le cas d'une capsule cardiaque). L'élément déformable 42 comprend une surface rigide 44 sur laquelle s'exerce la pression, et qui est reliée au reste du corps par l'intermédiaire d'un soufflet 46 déformable sous l'effet de la sollicitation externe à laquelle est soumise la surface rigide 44.

Dans l'exemple de la Figure 3, cet ensemble surface/soufflet 44, 46 est disposé sur une face d'extrémité axiale de la capsule 40, qui présente une forme générale cylindrique. Les dimensions sont typiquement de l'ordre de 6 mm de diamètre pour une longueur de 20 mm, soit un très faible volume de l'ordre de 0,5 cm³.

Dans l'exemple de la Figure 4, il est prévu deux ensembles déformables 42 disposés sur des faces latérales du corps 40 de la capsule, les surfaces rigides 44, reliées au bloc 40 par le soufflet 46, étant des surfaces parallèles entre elles et à l'axe principal de la capsule. Cette configuration permet notamment de dédoubler le système de récupération d'énergie ; elle libère en outre les deux extrémités axiales de la capsule, ce qui peut avoir son importance notamment pour y placer une vis d'ancrage sans que le système de récupération d'énergie ne fasse obstacle à cette configuration.

Dans l'un ou l'autre cas, le corps 40 avec son élément déformable 42 sont avantageusement réalisés sous forme monobloc, par exemple en titane évaporé ou électrodéposé sur un mandrin soluble.

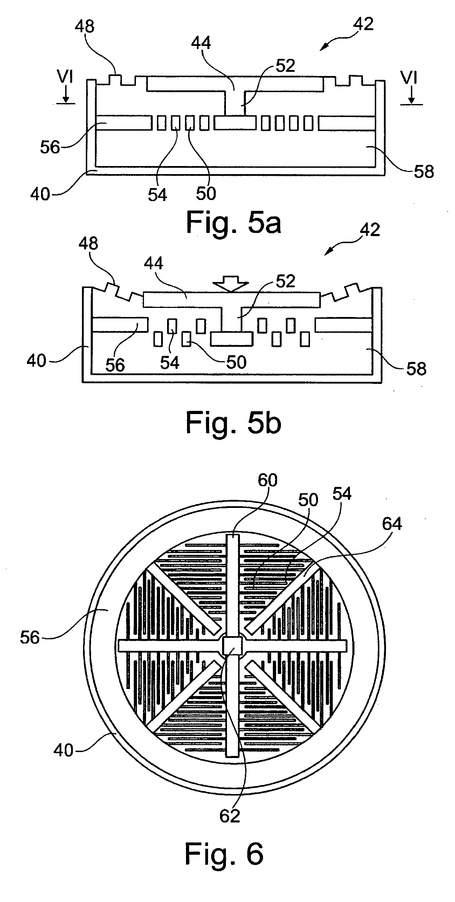

Les Figures 5a, 5b et 6 correspondent à un premier mode de réalisation d'une capsule à récupération d'énergie par voie électrostatique, décrit ici pour illustrer le principe du transducteur électrostatique à condensateur variable.

Dans ce premier mode de réalisation, l'élément déformable 42 comprend une surface rigide plane 44 couplée au corps 40 de la capsule par un élément élastique de liaison 48 réalisé sous forme d'ondulations périphériques formées autour de la surface rigide 44. La surface 44, qui est mobile sous les effets des variations de pression du milieu environnant, est reliée à une série de premières électrodes de condensateur 50 via l'élément de couplage 52, qui est ici simplement une tige. Comme on peut le voir notamment sur la Figure 4, les électrodes 50 sont avantageusement configurés sous la forme de peignes, réalisés par exemple par des techniques classiques de photogravure. Le dispositif comporte par ailleurs des secondes électrodes 54, par exemple réalisées sous la forme de contre-peignes interdigités avec les peignes des électrodes 50 (cf. Figure 6), reliés au corps 40 par un support périphérique 56. L'ensemble formé par ces électrodes 50, 54 est enfermé dans le volume étanche 58 constitué par le corps 40 fermé par l'élément déformable 42.

On dispose ainsi d'un transducteur qui peut être modélisé par un condensateur à capacité variable comprenant :
- une première électrode, suspendue, constituée par les peignes 50 qui sont mécaniquement et électriquement réunis par les bras 60 et le support central 62 relié à la surface mobile 44 ;
- une deuxième électrode, fixe, constituée par les contre-peignes 54 réunis mécaniquement et électriquement ensemble par les bras fixes 64 eux-mêmes solidaires du corps 40 via le support annulaire 56 ; et
- un intervalle diélectrique, défini entre les deux électrodes.

Avec les peignes et contre-peignes interdigités illustrés Figure 6, en cas d'enfoncement de l'élément déformable 42, l'entrefer et le chevauchement dans le plan des peignes restent constants, mais le chevauchement vertical change au cours du mouvement. La capacité est maximale quand les deux structures (peignes et contre-peignes) sont verticalement au même niveau, et elle est minimale (voisine de zéro) lorsque la structure mobile (les peignes suspendus 50) se sont déplacés d'une distance égale à leur épaisseur (comme illustré Figure 5b), ayant ainsi rendu quasiment nulles les surfaces en regard des peignes avec les contre-peignes. Concrètement, lorsqu'une pression externe s'exerce sur la surface mobile 44, par exemple au moment de la systole dans le cas d'une capsule immergée dans un milieu sanguin, la variation de pression produit un enfoncement de cette surface 44 vers l'intérieur de la capsule, comme illustré Figure 5b. Les peignes 50 de l'électrode mobile vont alors s'éloigner des peignes fixes 54 de l'électrode fixe et produire une variation de la capacité du condensateur, en l'occurrence une diminution de cette capacité du fait de la diminution des surfaces en vis-à-vis des électrodes fixe et mobile et de l'augmentation de l'intervalle diélectrique entre ces surfaces.

Si le condensateur avait été au préalable préchargé, la diminution de la capacité du condensateur va produire un surcroît d'énergie qui pourra être déchargé par des circuits appropriés vers un module de stockage, et permettre ainsi, à chaque cycle systolique, de récupérer une quantité d'énergie qui sera à terme suffisante pour assurer un fonctionnement permanent des circuits électroniques de la capsule sans apport supplémentaire d'énergie.

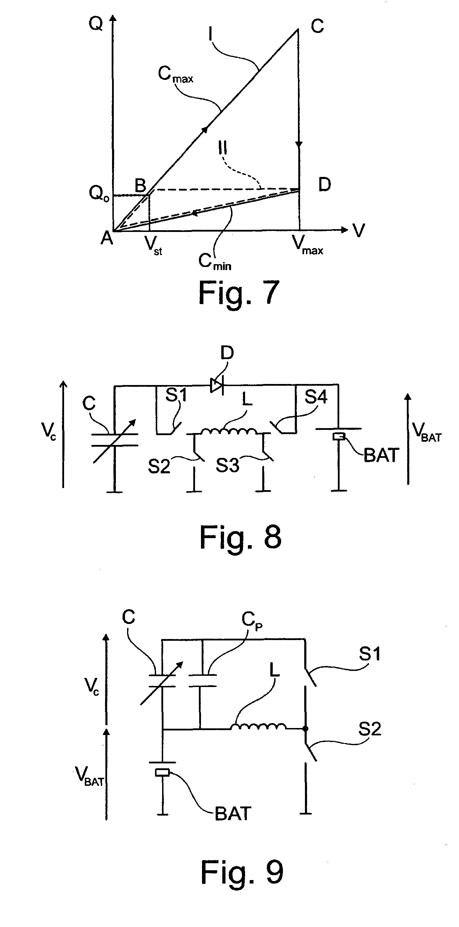

La précharge du condensateur peut être réalisée par des circuits électriques spécifiques, décrits ci-dessous en référence aux figures 7 à 9.

En variante, elle peut être réalisée par des éléments piézoélectriques annexes, qui lors des premières variations de pression vont se déformer et générer une tension préchargeant le condensateur lors de sa mise en service, selon une technique décrite notamment par Khbeis et al., Design of a Hybrid Ambient Low Frequency, Low Intensity Vibration Energy Scavenger, The Sixth International Workshop on Micro and Nanotechnology for Power Generation and Energy Conversion Applications, Berkeley, 2006, ou encore dans le FR 2 896 635 A1.

Dans une autre variante encore, la précharge peut être évitée en ayant une structure d'électret sur une des faces du condensateur, ces électrets générant d'eux-mêmes le champ électrique nécessaire. Cette technique est décrite notamment dans l'article cité de Peano Tambosso cité en introduction, ou encore par Sakane et al., The Development of a High-Performance Perfluorinated Polymer Electret and its Application to Micro Power Generation, Journal of Micromechanics and Microengineering, Vol. 18, pp.1-6, 2008.

Les Figures 7 à 9 explicitent la manière de récupérer l'énergie grâce à la variation de capacité du condensateur.

On va décrire deux techniques de récupération, respectivement à tension constante et à charge constante.

Le diagramme de la Figure 7 illustre deux caractéristiques charge/tension pour un cycle complet de charge/décharge du condensateur variable.

La caractéristique 1 correspond à un cycle ACDA à tension constante. Le condensateur est tout d'abord chargé à la tension maximale Vₘₐₓ (segment AC), pendant que la capacité est maximale (C = Cₘₐₓ), cette charge étant opérée en un temps suffisamment court (typiquement inférieur à la microseconde) pour que la capacité puisse être considérée comme constante.

Lors du déplacement de l'élément mobile, la capacité va se réduire de Cₘₐₓ à Cₘᵢₙ. La tension étant (par hypothèse) constante et maintenue à Vₘₐₓ, la caractéristique suit le segment CD. C'est pendant cette phase que l'énergie accumulée dans le condensateur est transférée au module de stockage. La charge résiduelle Q₀ est ensuite récupérée en suivant le segment DA, avec C = Cₘᵢₙ. L'énergie totale récupérée correspond à l'aire du cycle I soit ½ (Cₘₐₓ - Cₘᵢₙ)Vₘₐₓ².

Dans le cas d'une conversion à charge constante (caractéristique II suivant le chemin ABDA), le condensateur est initialement chargé à une tension de départ Vₛₜ, avec une capacité maximale C = Cₘₐₓ (segment AB). Le circuit est ensuite laissé ouvert (charge constante Q₀) durant le mouvement des électrodes du condensateur, qui fait passer la capacité de sa valeur maximale Cₘₐₓ à sa valeur minimale Cₘᵢₙ (segment BD), la tension croissant jusqu'à sa valeur maximale Vₘₐₓ pour satisfaire l'équation Q = CV. La charge est ensuite restituée (segment DA), de la même manière que précédemment. L'énergie totale récupérée est égale à l'aire du cycle II, soit ½(Cₘₐₓ - Cₘᵢₙ)VₛₜVₘₐₓ. Cette valeur est, pour une même tension maximale Vₘₐₓ, inférieure à celle de la solution à tension constante (caractéristique I) ; en revanche, cette solution peut procurer d'autres avantages, notamment la possibilité d'opérer avec une tension initiale faible. Il est également possible de prévoir un condensateur additionnel, monté en parallèle avec le condensateur variable C, pour augmenter l'énergie et se rapprocher ainsi des performances de la solution à tension constante.

La Figure 8 illustre de façon schématique un exemple de circuit pour la récupération d'énergie à tension constante.

Cette configuration de circuit est en elle-même connue, et pour plus de détails on pourra se référer par exemple à E. TorresetG. Rincon-Mora, Electrostatic Energy-Harvesting and Battery-Charging CMOS System Prototype, IEEE Transactions on Circuits and Systems I: Regular Papers, Vol. 56, n°9, 1938-1948, Sept. 2009.

Essentiellement, les quatre interrupteurs S1 à S4 sont initialement ouverts et le circuit surveille la tension aux bornes du condensateur C pour détecter le moment où celle-ci devient maximale. A ce moment, la phase de précharge est déclenchée, en commençant tout d'abord par charger l'inductance L (S1 et S3 fermés, S2 et S4 ouverts), puis en déchargeant cette inductance L dans le condensateur C (S1 et S3 ouverts, S2 et S4 fermés), le tout dans un temps très court par rapport à la variation de capacité du condensateur C. Les interrupteurs sont ensuite ouverts, et la diode D va venir fixer la tension aux bornes de C, en déchargeant le condensateur dans la batterie BAT, venant ainsi charger celle-ci.

La Figure 9 illustre un schéma de circuit de récupération d'énergie à charge constante. Ce circuit est également connu, et on pourra trouver plus de détails dans l'article précité de S. Meninger et al. Vibration-to-Electric Energy Conversion, IEEE Transactions on Very Large Scale Integration (VLSI) Systems, vol. 9, no. 1, pp. 64-76, 2001.

Essentiellement, la tension aux bornes des condensateurs C et Cₚ (un condensateur additionnel ajouté en parallèle à C pour augmenter l'énergie produite) est initialement nulle. Lorsque le circuit de contrôle détecte le maximum de capacité du condensateur C, S1 s'ouvre et S2 se ferme, ce qui charge l'inductance L, puis immédiatement après S1 se ferme et S2 s'ouvre, ce qui transfère l'énergie de L aux condensateurs C et Cₚ. Puis les deux interrupteurs S1 et S2 s'ouvrent et le condensateur C voit sa capacité diminuer sous l'effet des forces mécaniques, jusqu'à la valeur minimale Cₘᵢₙ. A cet instant, S1 est fermé et S2 restent ouverts, ce qui charge l'inductance L à partir de l'énergie accumulée dans les condensateurs C et Cₚ. Dès que la tension aux bornes de ces derniers s'annule, S1 s'ouvre et S2 se ferme, ce qui permet de transférer l'énergie recueillie de l'inductance L vers la batterie BAT.

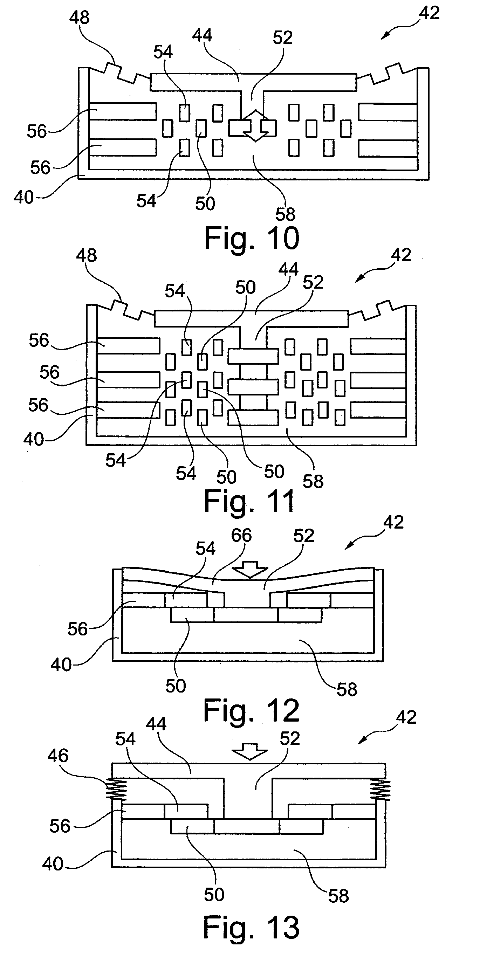

La Figure 10 illustre un deuxième mode de réalisation d'une capsule à récupération d'énergie par voie électrostatique, dans lequel l'électrode formée par le peigne mobile 50 est, au repos, positionnée entre deux contre-peignes fixes 54 superposés, de manière que le peigne mobile 50 vienne en regard de l'un ou l'autre des contre-peignes 54 suivant le sens de déplacement de la membrane. Ceci permet de récupérer l'énergie lorsque la membrane se déplace dans chacun des deux sens, par exemple pendant les deux phases de systole et de diastole dans le cas où la capsule est environnée d'un milieu sanguin.

La Figure 11 illustre un troisième mode de réalisation d'une capsule à récupération d'énergie par voie électrostatique, dans lequel le transducteur est constitué d'un empilement de structures telles que décrites en référence aux Figures 5 et 6, afin d'augmenter, la surface des électrodes en regard par la multiplication des ensembles peignes/contre-peignes, ce qui permet de maximiser la différence entre les capacités minimale Cₘᵢₙ et maximale Cₘₐₓ.

La Figure 12 illustre un quatrième mode de réalisation d'une capsule à récupération d'énergie par voie électrostatique, dans lequel l'élément déformable 42 est constitué d'une membrane souple 66, fixée au boîtier 40 de la capsule à sa périphérie et portant en son centre la pièce 52 de liaison à l'électrode mobile 50.

La Figure 13 illustre un cinquième mode de réalisation d'une capsule à récupération d'énergie par voie électrostatique, dans lequel l'élément déformable 42 est constitué d'un élément mobile rigide 44 s'étendant d'un bord à l'autre du boîtier 40, relié à celui-ci par un élément élastique 46 en forme de soufflet en lieu et place des ondulations périphériques 48 des modes de réalisation de Figures 10 et 11. Cette configuration permet en particulier d'augmenter à la fois le débattement de l'élément mobile 44, et donc celui de l'électrode mobile, et la surface de l'élément mobile 44 rigide sur lequel s'exerce la pression externe, avec augmentation corrélative de la force exercée au centre de cet élément.

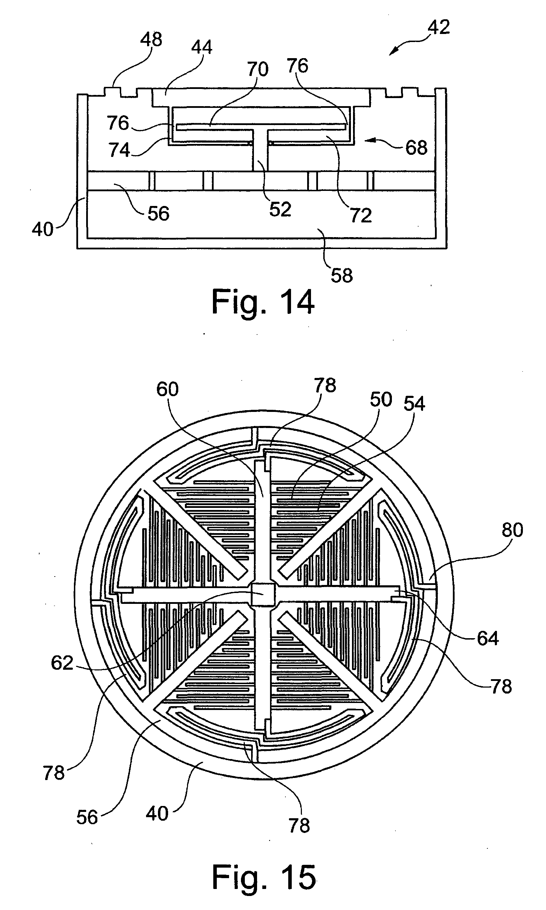

La Figure 14 illustre le perfectionnement apporté par la présente invention, qui est applicable indifféremment aux divers modes de réalisation décrits plus haut.

Il s'agit de pallier l'un des problèmes de la récupération des efforts exercés par les variations de pression sanguine, qui est la variation de la pression atmosphérique. En effet, l'intérieur de la capsule étant strictement étanche et donc à pression constante (ajustée en usine lors de la fabrication), si la pression atmosphérique varie, le point d'équilibre au repos de l'élément déformable va se trouver décalé par rapport à la position de repos nominale.

La solution proposée à la Figure 14 consiste à remplacer le couplage rigide entre l'élément déformable 42 et l'électrode mobile par un couplage incorporant un système de filtre passe-haut mécanique 68 interposé entre l'élément déformable soumis à la pression extérieure et la structure électrostatique mobile. Ce filtre est par exemple constitué d'un piston 70 dont la tige 52 est reliée à l'électrode mobile, ce piston se déplaçant dans un fluide 72 tel que de l'air ou autre gaz enfermé dans une enceinte étanche 74. De la sorte, les déplacements lents de l'élément déformable 42 dus aux variations de pression atmosphérique ne seront pas transférés à l'électrode mobile suspendue, le fluide pouvant s'écouler de part et d'autre du piston au travers d'orifices par exemple microstructurés ou par un jeu périphérique calibré 76 de manière à rétablir l'équilibre des pressions. En revanche, lors des variations rapides du cycle cardiaque diastole-systole, ces variations seront intégralement transmises à l'électrode suspendue, qui peut donc jouer complètement le rôle qui lui a été assigné.

La Figure 15 illustre un exemple de réalisation sous forme monolithique de la structure peignes/contre-peignes par des techniques de photogravure.

En effet, l'une des difficultés de conception des structures interdigitées à peignes et contre-peignes est d'obtenir un intervalle diélectrique le plus faible possible, afin de maximiser la capacité, tout en gardant une tolérance suffisante pour éviter que les peignes ne viennent en contact, qu'ils soient instables sous l'effet des forces électrostatiques mises en jeu si la rigidité transversale des doigts est trop faible, et qu'il survienne un claquage entre les électrodes si le champ électrique est trop intense.

Le dispositif présenté dans les divers modes de réalisation exposés plus haut (qui n'ont en eux-mêmes pas de caractère limitatif toutefois), avec un chevauchement hors plan variable, se prête avantageusement à une réalisation par des techniques classiques de microfabrication, en elles-mêmes connues lorsqu'il s'agit de réaliser des dispositifs de type peignes électrostatiques.

Les peignes 50 et contre-peignes 54 peuvent ainsi être réalisés simultanément sur une même tranche d'un substrat typiquement en silicium, fortement dopé pour être conducteur. La séparation des peignes pour former l'intervalle diélectrique peut être réalisée par gravure profonde du silicium par une technique de type DRIE (*Deep Reactive Ion Etching,* gravure ionique réactive profonde), ce qui permet par exemple d'obtenir des intervalles inférieurs à 10 µm sur une épaisseur de tranche de l'ordre de 300 à 500 µm.

Avec des intervalles aussi faibles que 10 pm, pour que l'intervalle entre les peignes reste constant et pour éviter que ces derniers ne se touchent, l'alignement et l'assemblage de deux structures indépendantes de peignes est difficile. Pour pallier cette difficulté, la structure peut être réalisée sur une tranche de type SOI (*Silicon On Insulator,* silicium sur isolant) dont le substrat est structuré de manière à former, comme illustré Figure 15, les peignes et contre-peignes 50, 54 proprement dits et leurs éléments supports communs 60, 64, et où la couche supérieure (active) de la tranche est structurée de manière à former des ressorts 78 très larges et peu épais entre chacun des supports 64 de la structure mobile et l'anneau périphérique 80 lié au corps 40 de la capsule.

Ces ressorts, du fait de leur configuration, vont présenter une rigidité importante dans le plan contenant la structure mobile suspendue des peignes 50, et limiter très fortement les déplacements transversaux, typiquement à moins de 1 µm. Ces éléments assurent donc, outre une fonction de support élastique dans le sens axial, une fonction de guidage et de centrage dans le plan transversal, garantissant ainsi un intervalle diélectrique sensiblement constant. Du fait de la très faible épaisseur de ces ressorts 78, ceux-ci vont être très souples dans la direction verticale (axiale), ce qui permet donc à l'élément déformable 42 de la capsule et à l'électrode suspendue constituée des peignes 50 de se déplacer axialement sans difficulté et sans ajout significatif de raideur.

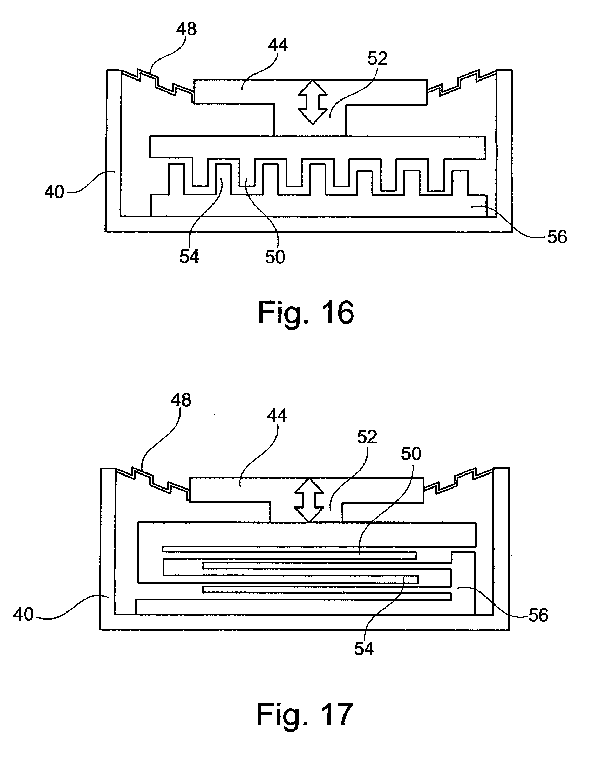

Les Figures 16 et 17 illustrent deux variantes de réalisation de la structure de condensateur, adaptées notamment à l'utilisation d'armatures porteuses d'électrets, comme cela est décrit dans les articles précités de Peano et al. et Sakane et al. Dans ce cas, les électrodes sont avantageusement configurées avec chevauchement dans le plan (Figure 16) ou bien avec un intervalle diélectrique variable (Figure 17). Le reste de la structure du transducteur est identique à ce qui a pu être décrit, selon diverses variantes, aux Figures 5 et 10-14.

## Revendications

1. Une capsule intracorporelle autonome, comportant un corps (40) et, à l'intérieur de ce corps, des circuits électroniques (26-36) et des moyens d'alimentation électrique comprenant :
- un transducteur de récupération d'énergie, apte à convertir une sollicitation physique extérieure appliquée à la capsule en une grandeur électrique, ce transducteur comprenant :
· une première électrode de condensateur (50), couplée à un élément mobile d'actionnement recevant ladite sollicitation physique extérieure,
ledit élément mobile d'actionnement étant essentiellement dépourvu de masse oscillante et comprenant une surface déformable (42), formée sur l'extérieur du corps de la capsule et apte à être déformée alternativement dans un sens et dans l'autre sous l'effet des variations de pression dans le milieu environnant la capsule, et
· une seconde électrode de condensateur (54), montée sur un support (56) relié à une région du corps autre que l'élément mobile d'actionnement,
les deux électrodes présentant des surfaces en vis-à-vis séparées par un intervalle diélectrique définissant ensemble un condensateur (C), et ladite sollicitation physique produisant une modification corrélative desdites surfaces en vis-à-vis et/ou dudit intervalle diélectrique avec variation corrélative de la capacité dudit condensateur ; et
- un module de stockage et de gestion d'énergie, alimenté par le transducteur de récupération d'énergie sous l'effet d'une diminution des surfaces en vis-à-vis et/ou d'une augmentation de l'intervalle diélectrique du condensateur,
capsule **caractérisée en ce que** la surface déformable (42) est couplée à la première électrode avec interposition d'un élément amortisseur mécanique (68) formant filtre passe-haut à l'égard des variations de pression dans le milieu environnant la capsule.

2. La capsule de la revendication 1, dans laquelle la surface déformable (42) comprend une surface rigide (44) couplée à la première électrode et un organe élastiquement déformable (46 ; 48) de liaison de cette surface rigide au reste du corps.

3. La capsule de la revendication 1, dans laquelle la surface déformable (42) comprend une membrane (66) couplée à la première électrode dans une région de plus grande déformation de celle-ci.

4. La capsule de la revendication 1, dans laquelle les première et seconde électrodes sont réalisées sous forme de peignes et contre-peignes inter-digités.

5. La capsule de la revendication 1, dans laquelle la première électrode est également couplée au corps de la capsule par un support élastiquement déformable (78) formant ressort de guidage.

6. La capsule de la revendication 1, comprenant en outre des moyens (L, D, S1, S2, S3, S4 ; L, S1, S2) aptes à précharger le condensateur lorsque sa capacité est maximale, et à décharger celui-ci en transférant son énergie vers les moyens de stockage (BAT) lorsque diminue cette capacité sous l'effet d'une diminution des surfaces en vis-à-vis et/ou d'une augmentation de l'intervalle diélectrique du condensateur.

## Patentansprüche

1. Autonome intrakorporale Kapsel, die einen Körper (40) und in diesem Körper elektronische Schaltungen (26-36) sowie Mittel zur elektrischen Versorgung enthält, die umfasst:
- einen Wandler zur Energierückgewinnung, der dafür ausgelegt ist, einen äußeren physikalischen Zwang, der auf die Kapsel ausgeübt wird, in eine elektrische Größe umzusetzen, wobei dieser Wandler Folgendes umfasst:
· eine erste Kondensatorelektrode (50), die mit einem beweglichen Betätigungselement gekoppelt ist, das den äußeren physikalischen Zwang aufnimmt,
wobei das bewegliche Betätigungselement im Wesentlichen keine oszillierende Masse besitzt und eine verformbare Oberfläche (42) aufweist, die außerhalb des Körpers der Kapsel gebildet ist und dafür ausgelegt ist, unter der Wirkung von Druckänderungen in dem die Kapsel umgebenden Milieu abwechselnd in einer Richtung und in einer anderen Richtung verformt zu werden, und
· eine zweite Kondensatorelektrode (54), die auf einem Träger (56) montiert ist, der mit einem Bereich des Körpers mit Ausnahme des beweglichen Betätigungselements verbunden ist,
wobei die zwei Elektroden gegenüberliegende Oberflächen aufweisen, die durch einen dielektrischen Spalt getrennt sind und zusammen einen Kondensator (C) definieren, und der physikalische Zwang eine korrelative Modifikation dieser gegenüberliegenden Oberflächen und/oder des dielektrischen Spalts mit einer damit korrelierten Änderung der Kapazität des Kondensators erzeugt; und
- ein Modul zum Speichern und Steuern von Energie, das durch den Wandler zur Energierückgewinnung unter der Wirkung einer Verringerung der gegenüberliegenden Oberflächen und/oder einer Erhöhung des dielektrischen Spalts des Kondensators versorgt wird,
wobei die Kapsel **dadurch gekennzeichnet ist, dass** die verformbare Oberfläche (42) mit der ersten Elektrode unter Einfügung eines mechanischen Dämpfungselements (68) gekoppelt ist, das ein Hochpassfilter in Bezug auf Druckänderungen in dem die Kapsel umgebenden Milieu bildet.

2. Kapsel nach Anspruch 1, wobei die verformbare Oberfläche (42) eine starre Oberfläche (44), die mit der ersten Elektrode gekoppelt ist, und ein elastisch verformbares Organ (46; 48) für die Verbindung dieser starren Oberfläche mit dem Rest des Körpers umfasst.

3. Kapsel nach Anspruch 1, wobei die verformbare Oberfläche (42) eine Membran (66) umfasst, die mit der ersten Elektrode in einem Bereich hiervon mit größerer Verformung gekoppelt ist.

4. Kapsel nach Anspruch 1, wobei die erste und die zweite Elektrode in Form von interdigitalen Kämmen und Gegenkämmen verwirklicht sind.

5. Kapsel nach Anspruch 1, wobei die erste Elektrode außerdem mit dem Körper der Kapsel durch einen elastisch verformbaren Träger (78), der eine Federführung bildet, gekoppelt ist.

6. Kapsel nach Anspruch 1, die außerdem Mittel (L, D, S1, S2, S3, S4; L, S1, S2) umfasst, die den Kondensator im Voraus aufladen können, wenn seine Kapazität maximal ist, und die diesen entladen können, indem sie seine Energie zu den Speichermitteln (BAT) übertragen, wenn diese Kapazität unter der Wirkung einer Verkleinerung der gegenüberliegenden Oberflächen und/oder einer Erhöhung des dielektrischen Spalts des Kondensators abnimmt.

## Claims

1. An autonomous intracorporeal capsule, including a body (40) and, inside this body, electronic circuits (26, 36) and electric supply means comprising:
- an energy harvesting transducer, adapted to convert an external physical stress applied to the capsule into an electrical quantity, said transducer comprising:
· a first capacitor electrode (50), coupled to a mobile actuating element receiving said external physical stress,
said mobile actuating element having essentially no oscillating mass and comprising a deformable surface (42), formed on the outside of the capsule's body and adapted to be deformed alternately in one direction and the other under the effect of the pressure variations in the environment surrounding the capsule, and
· a second capacitor electrode (54), mounted on a support (56) connected to a region of the body other than the mobile actuating element,
the two electrodes having opposite surfaces separated by a dielectric interval defining together a capacitor (C), and said physical stress producing a correlative modification of said opposite surfaces and/or said dielectric interval with a correlative variation of the capacitance of said capacitor ; and
- an energy storage and management module, supplied by the energy harvesting transducer under the effect of a decrease of the opposite surface areas and/or an increase of the dielectric interval of the capacitor,
the capsule being **characterized in that** the deformable surface (42) is coupled to the first electrode with interposition of a mechanical damping element (68) forming a high-pass filter regarding the pressure variations in the environment surrounding the capsule.

2. The capsule of claim 1, wherein the deformable surface (42) comprises a rigid surface (44) coupled to the first electrode and an elastically deformable member (46 ; 48) for connecting this rigid surface to the remaining of the body.

3. The capsule of claim 1, wherein the deformable surface (42) comprises a membrane (66) coupled to the first electrode in a region of greatest deformation of the latter.

4. The capsule of claim 1, wherein the first and second electrodes are made as interdigitated combs and counter-combs.

5. The capsule of claim 1, wherein the first electrode is also coupled to the capsule's body through an elastically deformable support (78) forming a guiding spring.

6. The capsule of claim 1, further comprising means (L, D, S1, S2, S3, S4; L, S1, S2) adapted to pre-charge the capacitor when its capacity is maximal, and to discharge it by transferring its energy towards the storage means (BAT) when this capacity decreases under the effect of a decrease of the opposite surface areas and/or an increase of the dielectric interval of the capacitor.
